# EUROPEAN PATENT SPECIFICATION

(11) **EP 1 550 424 B1**
(45) Date of publication and mention of the grant of the patent: **11.06.2014**
(21) Application number: 03791256.5
(22) Date of filing: 22.08.2003
(51) Int. Cl.: A61F 13/496, A61F 13/15

(54) **UNDERPANTS TYPE DISPOSABLE WEARING ARTICLE**
TRAGBARER EINWEGARTIKEL VOM HÖSCHENTYP
SOUS-VETEMENT JETABLE

(30) Priority: 30.08.2002 JP 2002255672
(43) Date of publication of application: 06.07.2005
(73) Proprietor: UNI-CHARM CO., LTD., Kawanoe, Ehime 799-0111 (JP)
(72) Inventor: SHIMOE, Nariaki, c/o Tech. Ctr, UNI-CHARM CO., LTD, Mitoyo-gun Kagawa 769-1602 (JP); OTSUBO, Toshifumi, c/o Tech. Ctr, UNI-CHARM CO LTD, Mitoyo-gun, Kagawa 769-1602 (JP)
(74) Representative: Sperling, Rüdiger
(86) International application number: PCT/JP2003/010667
(87) International publication number: WO 2004/019845

(56) References cited:
- EP-A1- 1 197 195
- EP-A2- 0 990 434
- JP-A- 9 099 006
- JP-A- 11 299 829
- JP-A- 2000 107 225

## Description

The present invention relates to a pull-on disposable wearing article for absorption and containment bodily discharges.

### RELATED ART

EP 0 990 434 A2 discloses a disposable pull-on diaper having in any one of front and rear waist regions of the diaper second elastic members disposed between the core and the backsheet to extend under tension circumferentially of the waist region and secured to the inner surface of the backsheet only at their portions extending outward beyond transversely opposite side edges.

Japanese Patent Application Publication No. 1997-56747A discloses a pull-on disposable wearing article comprising a liquid-pervious topsheet facing a wearer's body, a liquid-impervious backsheet facing away from the wearer's body and a liquid-absorbent core interposed between these top- and backsheets wherein front and rear waist regions are connected to each other in vicinities of lateral margins of waist's opposite lateral zones by means of a plurality of heat-sealing spots intermittently arranged in a longitudinal direction so as to form a waist-hole and a pair of leg-holes.

This wearing article is provided with a plurality of waist surrounding elastic members extending in a waist circumferential direction and attached to a waist's upper end portion in the front waist region so as to be contractible in a waist-circumferential direction. Between the waist surrounding elastic members and the leg-holes, the wearing article is further provided with a plurality of auxiliary elastic members extending in the waist-circumferential direction so as to be contractible in the waist-circumferential direction. The leg-holes' peripheral portions are also provided with a plurality of leg surrounding elastic members, respectively, so as to be contractible in the leg-circumferential direction.

The waist surrounding elastic members and the leg surrounding elastic members are interposed between the tops- and the backsheets and bonded to respective inner surfaces of these sheets by means of hot melt adhesive. Transversely opposite lateral zones of the respective auxiliary elastic members are also interposed between the top- and backsheets and secured to the inner surfaces of these sheets by means of a hot melt adhesive. Intermediate portions of the respective auxiliary elastic members extending across the core are interposed between the backsheet and the core and secured to the backsheet by means of a hot melt adhesive.

This wearing article has advantageous effects that a distance by which each pair of the adjacent auxiliary elastic members are spaced apart from each other is smaller in vicinities of longitudinally opposite ends of the core than in the other zone. Such a unique arrangement is effective to keep the wearing article in close contact with the wearer's waist in the vicinities of the longitudinally opposite ends of the core and thereby to prevent leakage of bodily discharges from occurring in vicinities of the ends of the core.

In the wearing article disclosed in the above-cited Publication, the auxiliary elastic members are secured to the top- and backsheets while these elastic members are stretched in the waist-circumferential direction at a predetermined stretching ratio, so contraction of the auxiliary elastic members in the waist-circumferential direction causes the top- and backsheets also to contract inward in the waist-circumferential direction. In this article, however, each of the auxiliary elastic members having been stretched can not restore its initial non-stretched dimension although these auxiliary elastic members contract inward in the waist-circumferential direction because contraction of the auxiliary elastic members is restricted by the presence of adhesive. Thus the regions of the top- and backsheets having the auxiliary elastic members therein are stretchable and contractible within a substantially narrower range than the range within which the auxiliary elastic members themselves are stretchable and contractible. This means that the range within which the auxiliary elastic members are stretchable and contractible can not be effectively utilized.

It is an object of the invention to provide a pull-on disposable wearing article improved so that the region of the article having the auxiliary elastic members therein may be stretchable and contractible within a substantially same range as the range within which the auxiliary elastic members themselves are stretchable and contractible.

### DISCLOSURE OF THE INVENTION

According to the present invention, there is provided a pull-on disposable wearing article generally comprising a base sheet defining front and rear waist regions opposed to each other and a crotch region extending between the waist regions and a liquid-absorbent panel attached to an inner side of the base sheet so as to extend over the crotch region further into the front and rear waist regions, the base sheet having a waist surrounding upper end portion lying outside longitudinally opposite ends of the panel and transversely opposite lateral zones lying outside transversely opposite side edges of the panel, front and rear waist regions defined by the base sheet Being connected to each other in the vicinity of lateral margins of the transversely opposite lateral zones to form a waist-hole and a pair of leg-holes, and the waist surrounding upper end portion being provided with waist surrounding elastic members so that the waist surrounding elastic members are contractible in a waist-circumferential direction.

The improvement according to the present invention further comprises the base sheet comprising a first sheet facing the panel and a second sheet lying outside the first sheet, and a plurality of auxiliary elastic members extending in the waist-circumferential direction across the panel are arranged between the waist surrounding elastic members and the leg-holes in at least one of the front and rear waist regions, the auxiliary elastic members being interposed between the first sheet and the second sheet and secured to the sheets so as to be contractible in the waist-circumferential direction; and a ratio of a dimension of a region of the base sheet in which the auxiliary elastic members are laid as the auxiliary elastic members have contracted to a dimension of the auxiliary elastic members in a non-stretched state in the waist-circumferential direction being in a range of 1.0 to 1.1.

The present invention includes the following embodiments.

Each of the auxiliary elastic members has longitudinal opposite end portions lying in the vicinity of the lateral margins of the transversely opposite lateral zones and secured to at least one of the first and second sheets and an intermediate portion extending between the opposite end portions and let free from the first and second sheets.

A stretching ratio of the auxiliary elastic members is in a range of 1.5 to 4.0.

A plurality of joining spots serving to join the first and second sheets are distributed between each pair of the auxiliary elastic members adjacent to each other so as to be arranged in a longitudinal direction in the vicinity of the opposite side edges of the panel.

The joining spots are distributed over a substantially entire area of the base sheet occupied by the panel.

Each of the auxiliary elastic members in a non-stretched state has a dimension as measured in the waist-circumferential direction is substantially equal to or slightly longer than a dimension of the panel lying in the front and rear waist regions as measured in the waist-circumferential direction.

### BRIEF DESCRIPTION OF THE DRAWINGS

Fig. 1 is a partially cutaway perspective view showing a disposable wearing article according to the invention;
Fig. 2 is a developed plan view showing the article of Fig. 1 with the front and rear waist regions disconnected from each other;
Fig. 3 is a sectional view taken along a line I-I in Fig. 2;
Fig. 4 is a sectional view taken along a line II-II in Fig. 2;
Fig. 5 is a partially cutaway perspective view showing another embodiment of the disposable wearing article not belonging to the invention;
Fig. 6 is a developed plan view showing the article of Fig. 5 with the front and rear waist regions being disconnected from each other;
Fig. 7 is a sectional view taken along a line III-III in Fig. 6; and
Fig. 8 is a sectional view taken along a line IV-IV in Fig. 6.

### DESCRIPTION OF THE BEST MODE FOR WORKING OF THE INVENTION

Detail of the pull-on disposable wearing article according to the present invention will be more fully understood from the description given hereunder with reference to the accompanying drawings.

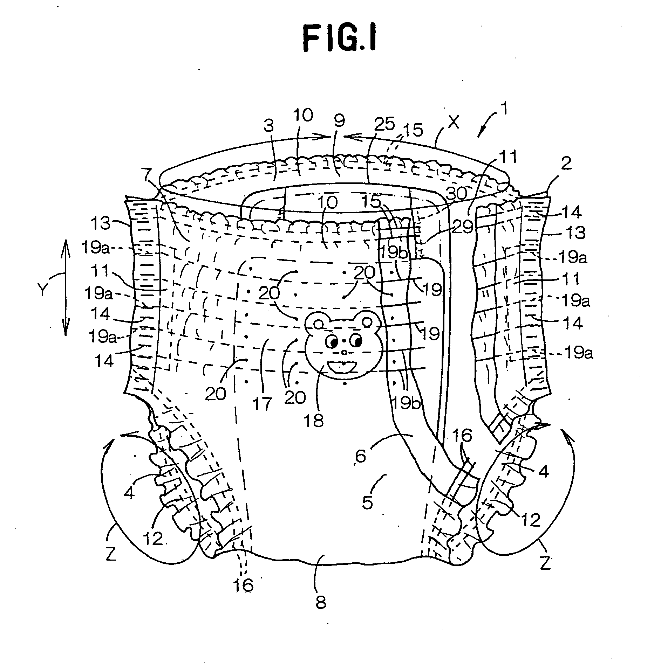

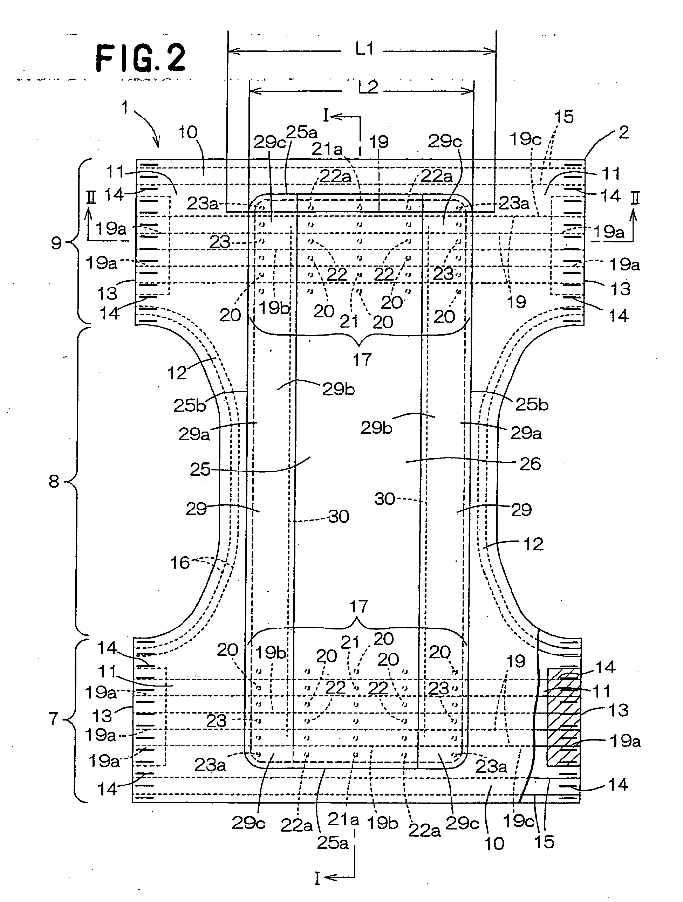

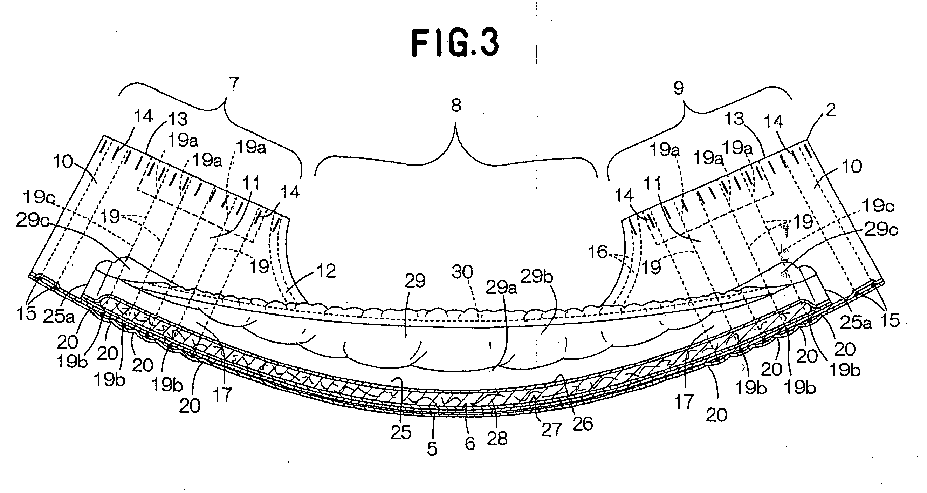

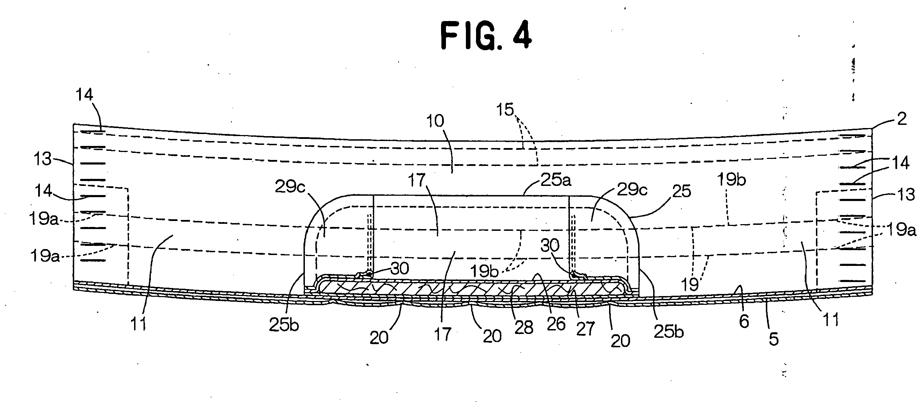

Fig. 1 is a partially cutaway perspective view showing a pull-on wearing article 1 according to a typical embodiment of the invention, Fig. 2 is a developed plan view showing the article 1 of Fig. 1 with front and rear waist regions disconnected from each other along transversely opposite lateral zones 11, Fig. 3 is a sectional view taken along a line I-I in Fig. 2, showing the article 1 slightly curved and Fig. 4 is a sectional view taken along a line II-II in Fig. 2. In Fig. 1, a waist-circumferential direction is indicated by an arrow X, a longitudinal direction is indicated by an arrow Y and a leg-circumferential direction is indicated by an arrow Z. In Fig. 2, chain double-dashed lines indicate an auxiliary elastic member 19 in a non-stretched state. Expression "inner surfaces of an inner sheet 6 (first sheet) and an outer sheet 5 (second sheet)" should be understood to be surfaces facing a panel 25 and expression "outer surfaces of these sheets 5, 6" should be understood to be surfaces facing away from the panel 25.

The article 1 comprises a base sheet 2 and the liquid-absorbent panel 25 attached to an inner side of the base sheet 2. The article 1 has a waist-hole 3 and a pair of leg-holes 4.

The base sheet 2 comprises the inner sheet 6 (first sheet) lying aside of the panel 25 and the outer sheet 5 (second sheet) lying outside the inner sheet 6. The base sheet 2 defines front and rear waist regions 7, 9 opposed to each other and a crotch region 8 extending between these waist regions 7, 9.

The base sheet 2 has a waist surrounding end portion 10 extending in the waist-circumferential direction outside longitudinally opposite ends 25a of the panel 25, the transversely opposite lateral zones 11 extending in the longitudinal direction outside transversely opposite side edges 25b of the panel 25 and peripheral edge portions 12 of the respective leg-holes 4 extending in the leg-circumferential direction outside the side edges 25b of the panel 25. In the base sheet 2, lateral margins 13 of the respective transversely lateral zones 11 are overlaid and joined together by means of a plurality of heat-sealing sites 14 intermittently arranged in the longitudinal direction in vicinities of the respective lateral margins 13.

Along the waist surrounding upper end portion 10, in the vicinities of the lateral margins 13 of the respective transversely opposite lateral zones 11 and along peripheral edge portions 12 of the respective leg-holes 4, the inner and outer sheets 6, 5 are overlaid together and these sheets 5, 6 having respective inner surfaces joined together. The waist surrounding upper end portion 10 is provided with a plurality of waist surrounding elastic members 15 so as to be contractible in the waist-circumferential direction. The waist surrounding upper end portion 10 is formed with a plurality of gathers as the waist surrounding elastic members 15 contract in the waist-circumferential direction. The peripheral edge portions 12 of the leg-holes 4 are provided with a plurality of leg surrounding elastic members 16 so as to be contractible in the leg-circumferential direction. The peripheral edge portions 12 of the leg-holes 4 are formed with a plurality of gathers as these leg surrounding elastic members 16 contract in the leg-circumferential direction. The waist surrounding elastic members 15 and the leg surrounding elastic members 16 are interposed between the inner sheet 6 and the outer sheet 5 and secured to these sheets 5, 6.

In the front waist region 7, a zone 17 of the outer sheet 5 occupied by the panel 25 is provided with an illustration of bear cub 18 (indication element). This illustration is printed on the outer surface of the outer sheet 5. So far as being recognizable from outside, it is possible to print the illustration 18 on the inner surface of the outer sheet 5 and it is also possible to print the illustration 18 either on the inner surface or on the outer surface of the inner sheet 6. The front and rear waist regions 7, 9 are provided with a plurality of auxiliary elastic members 19 extending across the panel 25 in the waist-circumferential direction so as to be contractible in the waist-circumferential direction.

The auxiliary elastic members 19 are interposed between the inner sheet 6 and the outer sheet 5 and spaced apart from one another by a given dimension in the longitudinal direction between the waist surrounding elastic members 15 and the leg-holes 4. Each of the auxiliary elastic members 19 has transversely opposite end portions 19a lying on the lateral margins 13 of the respective transversely opposite lateral zones 11 of the base sheet 2 and secured to the inner sheet 6 and the outer sheet 5 and an intermediate portion 19b lying between the opposite end portions 19a. The intermediate portion 19b is secured neither to the inner sheet 6 nor to the outer sheet 5, i.e., let free from these sheets 5, 6.

As will be seen in Fig. 2, each of the auxiliary elastic members 19 has a dimension L1 as measured in the waist-circumferential direction in a non-stretched state slightly longer than a dimension L2 of the panel 25 extending over the front and rear waist regions 7, 9 as measured in the waist-circumferential direction. The dimension L1 is preferably 1 to 5 mm longer than the dimension L2. Alternatively, the dimension L1 may be substantially equal to the dimension L2 of the panel 25.

Each of the auxiliary elastic members 19 is attached to the base sheet 2 in a stretched state. As the auxiliary elastic member 19 contracts in the waist-circumferential direction, the base sheet 2 contracts in the waist-circumferential direction. A ratio of a dimension of a region of the base sheet 2 to which the auxiliary elastic members 19 are laid as the auxiliary elastic members 19 have contracted to a dimension of the auxiliary elastic members 19 in a non-stretched state is preferably in a range of 1.0 to 1.1 as measured in the waist-circumferential direction. If the ratio exceeds 1.1, a range within which the region of the base sheet 2 is stretchable and contractible would be smaller than a range within which the auxiliary elastic members 19 are stretchable and contractible and therefore the range within which the auxiliary elastic members 19 are stretchable and contractible could not be sufficiently utilized.

The auxiliary elastic members 19 are attached to the base sheet 2 as the auxiliary elastic members 19 are stretched preferably at a stretching ratio of 1.5 to 4.0. If the stretching ratio is less than 1.5, the range within which the base sheet 2 is stretchable and contractible could not be sufficiently utilized and the range within which the base sheet 2 is stretchable and contractible would be thereby restricted. Consequently, the article 1 cannot be widely adjusted to the size of the wearer's waist. If the ratio exceeds 4.0, there is an anxiety that the transversely opposite end portions 19a of the respective auxiliary elastic members 19 might come off the sheets 5, 6.

The ratio of the dimension of the region of the base sheet 2 to which the auxiliary elastic members 19 are laid as the auxiliary elastic members 19 have contracted to the dimension of the auxiliary elastic members 19 in a non-stretched state was calculated in accordance with the following procedures:
(1) The auxiliary elastic members 19 are cut out together with the base sheet 2 from the article 1 to obtain a sample sheet for measurement.
(2) A dimension L3 of a portion of the sample sheet defined between a pair of heat-sealing spots 14 is measured when the portion as well as the auxiliary elastic member 19 have contracted in the waist-circumferential direction (except for the vicinities of the lateral margins 13 of the transversely opposite lateral zone 11 along which the heat-sealing sites 14 are intermittently arranged).
(3) After measurement of the dimension L3, the auxiliary elastic members 19 are cut between a pair of the heat-sealing sites 14 (specifically, in vicinities of the heat-sealing sites 14). Then an adhesive is rinsed out using a suitable organic solvent such as toluene to separate the sheets 5, 6 and the auxiliary elastic members 19 having been cut from one another. Thereafter the auxiliary elastic members 19 are dried and a dimension L4 of the auxiliary elastic members 19 in a non-stretched state is measured as viewed in the waist-circumferential direction.
(4) The dimension L3 is divided by the dimension L4 to calculate the ratio of the dimension L3 to the dimension L4.

In the front and rear waist regions 7, 9, the inner sheet 6 and the outer sheet 5 are joined together by means of a plurality of adhesive spots 20. These adhesive spots 20 are distributed between each pair of the adjacent auxiliary elastic members 19 at given intervals in the longitudinal direction as well as in the waist-circumferential direction. These adhesive spots 20 are provided in the form of dots and distributed in substantially entire area of the zone 17 of the front and rear waist regions 7, 9 occupied by the panel 25.

The adhesive spots 20 comprise an array of first adhesive spots 21 intermittently arranged in the longitudinal direction in a transversely middle zone of the panel 25, two arrays of second adhesive spots 22 intermittently arranged in the longitudinal direction on both sides of the array of first adhesive spots 21 and two arrays of third adhesive spots 23 intermittently arranged in the longitudinal direction in vicinities of the transversely opposite side edges 25b of the panel 25. Of the first and second adhesive spots 21, 22, the adhesive spots 21a, 22a at the uppermost positions of the front and rear waist regions 7, 9 are formed between the auxiliary elastic member 19c extending in the vicinities of the longitudinally opposite ends 25a of the panel 25 and these opposite ends of the panel 25. Of the third adhesive spots 23, the adhesive spots 23a at the uppermost positions of the front and rear waist regions 7, 9 are formed at cross-points of the longitudinally opposite ends 25a and the transversely opposite side edges 25b of the panel 25.

The number of the adhesive spots 20 arranged in the longitudinal direction is not limited to the number as illustrated. The pattern of the adhesive spots is not limited to the dot-pattern as illustrated but may be line-pattern or ribbon-pattern wherein each of the lines or ribbons extends in the waist-circumferential direction. The first and second adhesive dots are not essential so far as the outer sheet 5 and the inner sheet 6 are reliably joined together by means of the third adhesive spots 23.

The panel 25 comprises a liquid-pervious upper sheet 26 facing the wearer's body, a liquid-impervious lower sheet 27 facing away from the wearer's body and a liquid-absorbent core 28 interposed between the upper and lower sheets 26, 27 and secured to respective inner surfaces of these sheets 26, 27. The panel 25 presents a substantially rectangular planar shape and extends over the crotch region 8 of the base sheet 2 into the front and rear waist regions 7, 9. The panel 25 is provided in the vicinities of its opposite side edges 25b with a pair of substantially liquid-impervious side sheets 29.

In the panel 25, peripheral margins of the upper and lower sheets 26, 27 extending outward beyond the peripheral edge of the core 28 are overlaid and joined together. Over a substantially entire area of the panel 25 lying in the front and rear waist regions 7, 9 of the base sheet 2 is secured to the inner surface of the inner sheet 6 extending in the front and rear waist regions 7, 9 by means of the lower sheet 27.

Each of the side sheets 29 has a fixed side edge portion 29a secured to the panel 25 in the vicinities of the associated side edge 25b so as to extend in the longitudinal direction, a free side edge portion 29b extending parallel to the fixed side edge portion 29a in the longitudinal direction and longitudinally opposite fixed end portions 29c collapsed toward the transversely middle zone of the panel 25 and secured to the panel 25 in the vicinities of the respective end portions 25a of the panel 25 so as to be held in the collapsed state. The free side edge portion 29b is provided with an elastically stretchable member 30 attached thereto in a stretched state.

The core 28 comprises a mixture of fluff pulp and super-absorbent polymer particles or a mixture of fluff pulp, super-absorbent polymer particles and thermoplastic synthetic resin fibers, in both cases, compressed to a desired thickness. Thus the panel 25 has a stiffness higher than those of the inner sheet 6 and the outer sheet 5.

Preferably, the core 28 is entirely wrapped with a liquid-pervious sheet such as a tissue paper or a hydrophilic fibrous nonwoven fabric in order to prevent the core 28 from getting out of shape and/or to avoid falling off of the polymer particles. The polymer particles may be selected from the group consisting of starch-based polymer particles, cellulose-based polymer particles and synthetic polymer particles.

The elastic members 30 contract as the panel 25 curves in the longitudinal direction with the upper sheet 26 inside and thereupon the respective free side edge portions 29b of the side sheets 29 rise above the upper sheet 26 so as to form barriers adapted to prevent bodily discharges from leaking out beyond the opposite side edges 25b of the panel 25.

Joining of the inner and outer sheets 6, 5 to each other, attaching of the elastic members 15, 16, 19 to the inner and outer sheets 6, 5, joining of the inner sheet 6 and the lower sheet 27 to each other, joining of the upper and lower sheets 26, 27 to each other and securing of the core 28 to the upper and lower sheets 26, 27 are carried out using a hot melt adhesive (not shown).

In the article 1, each of the auxiliary elastic members 19 has its intermediate portion 19b secured neither to the inner sheet 6 nor to the outer sheet 5 and therefore so far as the intermediate portion 19b is concerned, contraction of the auxiliary elastic member 19 is not restricted by the presence of the adhesive. Consequently, it can be ensured that the region of the base sheet 2 in which the auxiliary elastic members 19 are laid is contracted at the ratio of 1.0 to 1.1 to the dimension of the auxiliary elastic members 19 in a non-stretched state. In this way, the region of the base sheet 2 in which the auxiliary elastic members 19 are laid is stretchable and contractible within a substantially same range as the range within which the auxiliary elastic members 19 are stretchable and contractible. As a result, the article 1 can be widely adjusted to the size of the wearer's waist.

In the article 1, the intermediate portions 19b of the respective auxiliary elastic members 19 are secured neither to the inner sheet 6 nor to the outer sheet 5, so the inner sheet 6 and the outer sheet 5 are not formed with a plurality of gathers except for the waist surrounding upper end portion 10 and the vicinities of the lateral margins 13 of the respective transversely opposite lateral zones 11. The absence of the gathers is effective to improve touch and appearance of these sheets 5, 6. In the region 17 of these sheets 5, 6 occupied by the panel 25, due to a stiffness of the panel 25, a contractile force of the auxiliary elastic members 19 is prevented from affecting the portions of the inner sheet 6 and the outer sheet 6 lying in the region 17 even when the auxiliary elastic members 19 contract in the waist-circumferential direction. Thus the outer sheet 5 is kept substantially flat and the illustration 18 of the bear cub displayed on this outer sheet 5 can be clearly recognized.

In the article 1, the dimension L1 of the auxiliary elastic members 19 in a non-stretched state in the waist-circumferential direction is 1 to 5 mm longer than the dimension of the panel 25 defined between its transversely opposite side edges 25b in the waist-circumferential direction. With this dimensioning, the panel 25 is not formed with gathers or wrinkles due to the contractile force of the auxiliary elastic members 19. This is for the reason that the auxiliary elastic members 19 do not contract in the waist-circumferential direction so that the dimension of the auxiliary elastic members 19 may be smaller than the dimension L2 of the panel 25 even when the auxiliary elastic members 19 contract in the waist-circumferential direction.

In the article 1, a plurality of adhesive spots 20 distributed between each pair of the adjacent auxiliary elastic members 19 serve to prevent the auxiliary elastic members 19 from shifting in the longitudinal direction. Furthermore, the article 1 is provided between the auxiliary elastic members 19c extending in the transverse direction in the vicinities of the longitudinally opposite ends 25a of the panel 25 and the respective longitudinally opposite ends 25a of the panel 25 with adhesive spots 21a, 22a, 23a. These adhesive spots 21a, 22a, 23a serve to prevent the auxiliary elastic members 19c from shifting outward beyond the longitudinally opposite ends 25a of the panel 25. In addition, the auxiliary elastic members 19c function to support the panel 25 in the vicinities of its longitudinally opposite ends 25a and thereby to prevent these opposite ends 25a of the panel 25 from protruding outward from the base sheet 2.

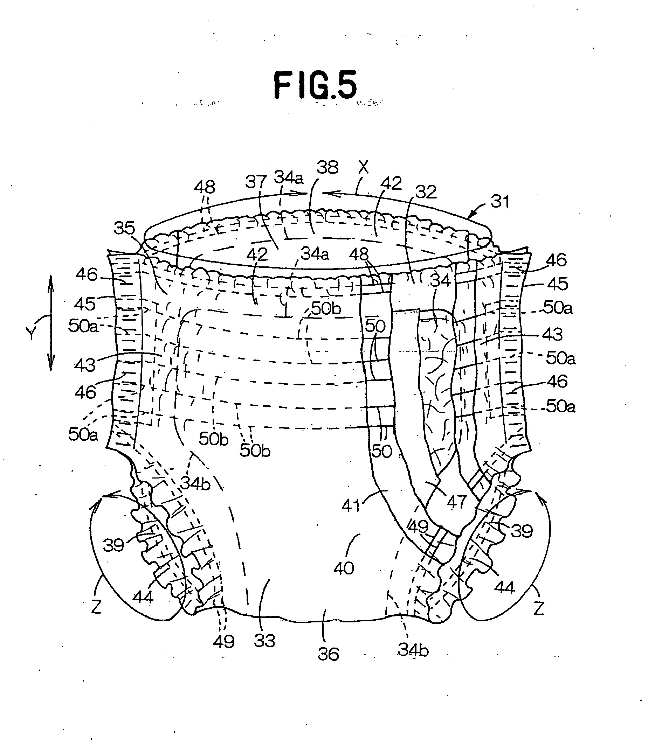

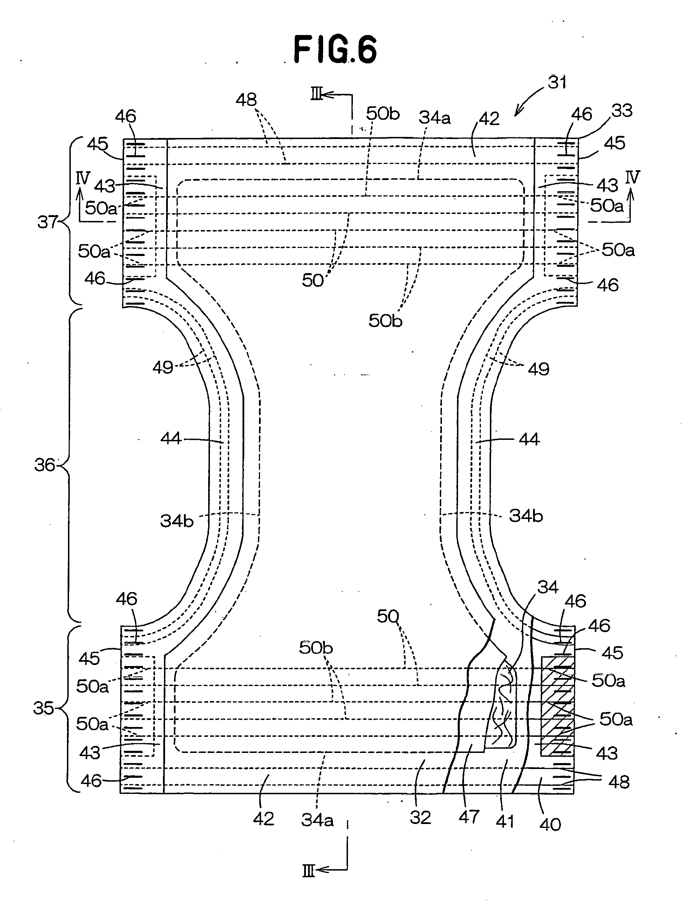

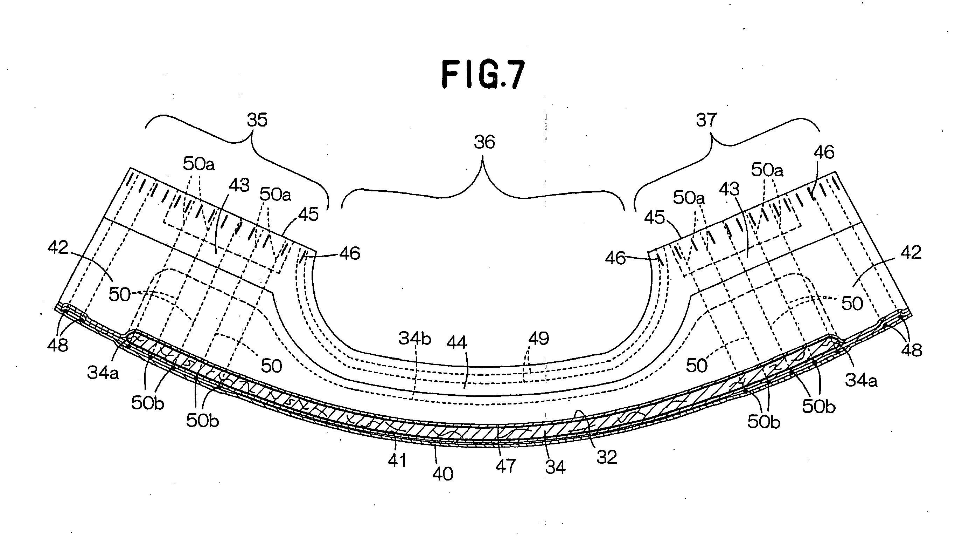

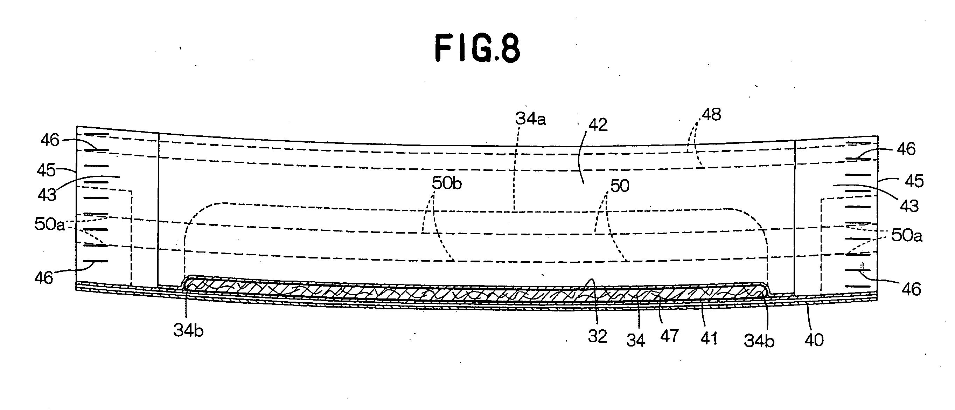

Fig. 5 is a partially cutaway perspective view showing a disposable wearing article 31 not according to the invention, Fig. 6 is a developed plan view showing the article 31 of Fig. 5 with the front and rear transversely opposite lateral zones 43 disconnected from one another, Fig. 7 is a sectional view taken along a line III-III in Fig. 6, showing the article 31 as slightly curving and Fig. 8 is a sectional view taken along a line IV-IV in Fig. 6. In Fig. 5, the waist-circumferential direction is indicated by the arrow X, the longitudinal direction is indicated by the arrow Y and the leg-circumferential direction is indicated by the arrow Y.

The article 31 comprises a liquid-pervious topsheet 32 facing the wearer's body, a liquid-impervious backsheet 33 (base sheet) and a liquid-absorbent core 34 (liquid-absorbent panel). The article 1 has front and rear waist regions 35, 37 and a crotch region 36 lying between these front and rear waist regions 35, 37.

The article 31 has a waist surrounding upper end portion 42 lying outside longitudinally opposite ends 34a of the core 34, transversely opposite lateral zones 43 lying outside transversely opposite side edges 34b of the core 34 and leg-holes' peripheral portions 44 lying outside the respective side edges 34b of the core 34. In the article 31, lateral margins 45 of the respective transversely opposite lateral zones 43 are overlaid and joined together by means of a plurality of heat-sealing sites 46 intermittently arranged in the longitudinal direction in vicinities of the respective lateral margins 45. The article 31 has a waist-hole 38 and a pair of leg-holes 39.

The backsheet 33 comprises an inner sheet 41 (first sheet) facing the core 34 and an outer sheet 40 (second sheet) lying outside the inner sheet 41. The outer sheet 40 and the inner sheet 41 are overlaid and joined together along the waist surrounding upper end portion 42, in the vicinities of the lateral margins 45 of the transversely opposite lateral zones 43 and along the leg-holes' peripheral portions 44.

The core 34 extends over the crotch region 36 into the front and rear waist regions 35, 37. Similarly to the core 28 shown in Fig. 1, the core 34 has a stiffness higher than those of the topsheet 32, the inner sheet 41 and the outer sheet 40. The core 34 is entirely wrapped with a tissue paper 47 and has the lower surface which is, substantially over its entire area, secured to the inner surface of the inner sheet 41 extending in the front waist region 35, the crotch region 36 and the rear waist region 37 with the tissue paper 47 therebetween.

A dimension of the topsheet 32 as measured in the waist-circumferential direction is smaller than those of the inner sheet 41 and the outer sheet 40 as measured in the waist-circumferential direction. A peripheral portion of the topsheet 32 extending outward beyond the peripheral edge of the core 34 is secured to the inner surface of the inner sheet 41.

A plurality of waist surrounding elastic members 48 extending in the waist-circumferential direction are attached to the waist surrounding upper end portion 42 so as to be contractible in the waist-circumferential direction. Similarly, a plurality of leg surrounding elastic members 49 extending in the leg-circumferential direction are attached to the respective leg-holes' peripheral portions 44 so as to be contractible in the leg-circumferential direction. Both the waist surrounding elastic members 48 and the leg surrounding elastic members 49 are interposed between the inner sheet 41 and the outer sheet 40 and secured to the sheets 40, 41.

The front and rear waist regions 35, 37 are provided with a plurality of auxiliary elastic members 50 extending in the waist-circumferential direction across the core 34 so as to be contractible in the waist-circumferential direction. These auxiliary elastic members 50 are interposed between the inner sheet 41 and the outer sheet 40 and arranged between the waist surrounding elastic members 48 and the leg-holes 39 so that the auxiliaryelastic members 50 adjacent to each other are spaced apart from each other by a given dimension in the longitudinal direction. Each of the auxiliary elastic members 50 has transversely opposite end portions 50a lying in the vicinities of the lateral margins 45 of the respective transversely opposite lateral zones 43 and secured to the inner sheet 41 and the outer sheet 40 and an intermediate portion 50b lying between the opposite end portions 50a and let free from both the outer sheet 40 and the inner sheet 41.

Each of the auxiliary elastic members 50 in a non-stretched state preferably has a substantially equal to a dimension of the core 34 or 1 to 5 mm longer than the dimension of the core 34 as measured in the waist-circumferential direction. A ratio of the dimension of the region of the backsheet 33 to which the auxiliary elastic members 50 are laid as the auxiliary elastic members 50 have contracted (i.e., the dimension L3) to the dimension of the auxiliary elastic members 50 in a non-stretched state (i.e., the dimension L4) is preferably in a range of 1.0 to 1.1 as measured in the waist-circumferential direction. The auxiliary elastic members 50 are attached to the backsheet 33 as these auxiliary elastic members 50 are stretched preferably at a ratio of 1.5 to 4.0. The ratio of the dimension of the region of the backsheet 33 to which the auxiliary elastic members 50 are laid as the auxiliary elastic members 50 have contracted to the dimension of the auxiliary elastic members 50 in a non-stretched state was calculated in accordance with the same procedures as taken for the article 1 of Fig. 1.

Joining of the inner sheet 41 and the outer sheets 40 to each other, attaching of the elastic members 48, 49, 50 to the inner sheet 41 and the outer sheets 40, and securing of the topsheet 32 and the core 34 to the inner sheet 41 are carried out using a hot melt adhesive (not shown).

In the article 31, each of the auxiliary elastic members 50 has its intermediate portion 50b secured neither to the inner sheet 6 nor to the outer sheet 5 and therefore so far as the intermediate portion 50b is concerned, contraction of the auxiliary elastic member 50 is not restricted by the presence of the adhesive. Consequently, it can be ensured that the region of the backsheet 33 in which the auxiliary elastic members 50 are laid is contracted at the ratio of 1.0 to 1.1 to the non-stretched dimension of the auxiliary elastic members 50 in a non-stretched state. In this way, the region of the backsheet 33 in which the auxiliary elastic members 50 are laid is stretchable and contractible within a substantially same range as the range within which the auxiliary elastic members 50 are stretchable and contractible. As a result, the article 1 can be widely adjusted to the size of the wearer's waist.

In the article 31, the outer and inner sheets 40, 41 extending in the front and rear waist regions 35, 37 undulate gently as the auxiliary elastic members 50 contract in the waist-circumferential direction. However, it is not likely that the outer and inner sheets 40, 41 might be formed with a plurality of fine gathers except for the waist surrounding upper end portion 42, the vicinities of the lateral margins 45 of the lateral zones 43 and the peripheral portions 44 of the leg-hole 39. This is for the reason that the intermediate portions 50b of the respective auxiliary elastic members 50 are not secured to the outer and inner sheets 40, 41. Furthermore, it is not likely that the auxiliary elastic members 50 might contract to a dimension smaller than the dimension of the core 34 as measured in the waist-circumferential direction and the core 34 might be formed with wrinkles due to a stretch stress of the auxiliary elastic members 50.

A stock material for the outer sheet 5, 40, the inner sheet 6, 41 and the lower sheet 27 may be selected from the group consisting of a substantially liquid-impervious hydrophobic fibrous nonwoven fabric, a breathable but liquid-impervious thermoplastic film, composite nonwoven fabric comprising two or more layers of hydrophobic fibrous nonwoven fabric laminated upon one another and a composite sheet comprising a hydrophobic fibrous nonwoven fabric and a breathable but liquid-impervious plastic film placed upon each other. A stock material for the upper sheet 26 and the topsheet 32 may be selected from the group consisting of a fibrous nonwoven fabric treated to be hydrophilic, a thermoplastic film having a plurality of perforations and a hydrophobic fibrous nonwoven fabric.

The nonwoven fabric may be selected from the group consisting of those obtained by spun lace-, needle punch-, melt blown-, thermal bond-, spun bond-, chemical bond- and air-through-processes. Component fibers of the nonwoven fabric may be selected from the group consisting of polyolefine-, polyester- and polyamide-based fibers and core-and-sheath type or side-by-side type conjugated fibers of polyethylene/polypropylene and polyethylene/polyester.

The pull-on disposable wearing article according to the invention has advantageous effects that the intermediate portions of the respective auxiliary elastic members are let free from the first and second sheets. This feature ensures that the ratio of the dimension of the region of the base sheet to which the auxiliary elastic members are laid as the auxiliary elastic members have contracted to the dimension of the auxiliary elastic members in a non-stretched state is held in a range of 1.0 to 1.1 as measured in the waist-circumferential direction. In this article, the region of the base sheet to which the auxiliary elastic members are laid is stretchable and contractible within a substantially same range as the range within which the auxiliary elastic members are stretchable and contractible. In this way, this article can be widely adjusted to the size of wearer' s waist. Except for the waist surrounding upper end portion, the vicinities of the lateral margins of the respective lateral zones and the peripheral portions of the leg-hole, there is no possibility that the base sheet might be formed with a plurality of fine gathers. Thus not only touch but also appearance of the base sheet can be improved.

With the article in which the first and second sheets are joined together by means of a plurality of bonding spots each formed between each pair of the auxiliary elastic members adjacent to each other so as to be arranged in the longitudinal direction, there is no anxiety that the auxiliary elastic members might shift in the longitudinal direction in the front and rear waist regions.

In the article according to the embodiment in which the dimension of the auxiliary elastic members in a non-stretched state as measured in the waist-circumferential direction is substantially equal to or slightly larger than the dimension of the region of the panel defined between the transversely opposite side edges of the panel, it is not likely that the auxiliary elastic members might contract to a dimension smaller than the dimension of the panel as measured in the waist-circumferential direction and thereby the panel might be formed with wrinkles due to the contractile force of the auxiliary elastic members even when the auxiliary elastic members contract in the waist-circumferential direction.

## Claims

1. A pull-on disposable wearing article (1) generally comprising a base sheet (2) defining front and rear waist regions (7, 9) opposed to each other and a crotch region (8) extending between said waist regions and a liquid-absorbent panel (25) attached to an inner side of said base sheet (2) so as to extend over said crotch region (8) further into said front and rear waist regions (7, 9), said base sheet (2) having a waist surrounding upper end portion (10) lying outside longitudinally opposite ends (25a) of said panel and transversely opposite lateral zones (11) lying outside transversely opposite side edges (25b) of said panel, front and rear waist regions defined by said base sheet being connected to each other in the vicinity of lateral margins (13) of said transversely opposite lateral zones (11) to form a waist-hole (38) and a pair of leg-holes (39), and said waist surrounding upper end portion (10) being provided with waist surrounding elastic members (15) so that said waist surrounding elastic members are contractible in a waist-circumferential direction, wherein:
said base sheet (2) comprises a first sheet (6) facing said panel (25) and a second sheet (5) lying outside said first sheet, and a plurality of auxiliary elastic members (19) extending in the waist-circumferential direction across said panel (25) are arranged between said waist surrounding elastic members and said leg-holes in at least one of said front and rear waist regions (7, 9), and said auxiliary elastic members (15) are interposed between said first sheet (6) and said second sheet (5) and secured to said sheets (5, 6) so as to be contractible in said waist-circumferential direction,
and each of said auxiliary elastic members (19) has transversely opposite end portions (19a) lying in the vicinity of said lateral margins (13) of said transversely opposite lateral zones and secured to at least one of said first and second sheets (6, 5) and an intermediate portion (19b) extending between said opposite end portions and let free from said first and second sheets,
a plurality of adhesive joining spots (20) serving to join said first and second sheets (6, 5) are distributed between each pair of said auxiliary elastic members (19) adjacent to each other at given intervals in the longitudinal direction as well as in the waist-circumferential direction,
said adhesive joining spots (20) are distributed in substantially entire area of each zone (17) of said front and rear waist regions (7, 9) occupied by said panel (25);
a ratio of a dimension of a region of said base sheet (2) in which said auxiliary elastic members (19) are laid as said auxiliary elastic members have contracted to a dimension of said auxiliary elastic members in a non-stretched state in said waist-circumferential direction is in a range of 1.0 to 1.1.

2. The wearing article according to Claim 1, wherein a stretching ratio of said auxiliary elastic members (19) is in a range of 1.5 to 4.0.

3. The wearing article according to Claim 1, wherein said plurality of adhesive joining spots (20) is arranged in said longitudinal direction in vicinities of said opposite side edges of said panel (25).

4. The wearing article according to any of claims 1 through 3, wherein each of said auxiliary elastic members (19) in a non-stretched state has a dimension as measured in said waist-circumferential direction is substantially equal to a dimension of said panel (25) lying in said front and rear waist regions as measured in said waist-circumferential direction

## Patentansprüche

1. Anziehbarer Wegwerf-Trageartikel (1), der im Allgemeinen eine Basislage (2), die vordere und hintere, sich gegenüber liegende Taillenbereiche (7, 9) und einen Schrittbereich (8) bestimmt, der sich zwischen den Taillenbereichen erstreckt, und ein flüssigkeitsabsorbierendes Panel (25), das auf einer Innenseite der Basislage (2) befestigt ist, so dass es sich über den Schrittbereich (8) weiter in den vorderen und hinteren Taillenbereich (7, 9) erstreckt, umfasst, wobei die Basislage (2) einen taillenumgebenden oberen Endabschnitt (10), der außerhalb sich longitudinal gegenüber liegender Enden (25a) des Panels liegt, und transversal gegenüber liegende laterale Bereiche (11), die außerhalb sich quer gegenüber liegender Seitenkanten (25a) des Panels liegen, aufweist, wobei die vorderen und hinteren durch die Basislage bestimmten Taillenbereiche miteinander nahe lateraler Ränder (13) der transversalen, sich gegenüber liegenden lateralen Bereiche (11) verbunden sind, um eine Taillenöffnung (38) und ein Paar Beinöffnungen (39) zu bilden, und der taillenumgebende obere Endabschnitt mit taillenumgebenden elastischen Elementen (15) versehen ist, so dass die taillenumgebenden elastischen Elemente in einer taillenumgebenden Richtung kontrahierbar sind, wobei:
die Basislage (2) eine erste Lage (6), die dem Panel (25) zuweist, und eine zweite Lage (5), die außerhalb der ersten Lage liegt, aufweist, und eine Vielzahl von elastischen Hilfselementen (19), die sich in der taillenumgebenden Richtung über das Panel (25) erstrecken, sind zwischen den taillenumgebenden elastischen Elementen und den Beinöffnungen zumindest in einem der vorderen oder hinteren Taillenbereiche (7, 9) angeordnet, und die elastischen Hilfselemente (15) sind zwischen der ersten Lage (6) und der zweiten Lage (5) eingefügt und an den Lagen (5, 6) befestigt, um in der taillenumgebenden Richtung kontrahierbar zu sein,
und jedes der elastischen Hilfselemente (19) weist sich transversal gegenüber liegende Endabschnitte (19a), die nahe der lateralen Ränder (13) der sich transversal gegenüber liegenden lateralen Bereiche liegen und an zumindest einer der ersten oder zweiten Lage (5, 6) befestigt sind, und einen Mittelabschnitt (19b) auf, der sich zwischen den sich gegenüberliegenden Endabschnitten erstreckt und von der ersten und zweiten Lage gelöst ist,
eine Vielzahl von klebenden Verbindungspunkten (20) zum Verbinden der ersten und zweiten Lagen (5, 6) zwischen jedem Paar der elastischen Hilfselemente (19) aneinander in einem bestimmten Abstand in Längsrichtung und in der taillenumgebenden Richtung angrenzend verteilt sind,
die klebenden Verbindungspunkte (20) im Wesentlichen in dem gesamten Bereich jedes Bereichs (17) der vorderen und hinteren Taillenbereiche (7, 9), die von dem Panel (25) eingenommen sind, verteilt sind;
ein Verhältnis der Größe eines Bereichs der Basislage (2), in dem die elastischen Hilfselemente (19) liegen, wenn die elastischen Hilfselemente kontrahiert sind, zu einer Größe der elastischen Hilfselemente in einem in der taillenumgebenden Richtung ungedehnten Zustand in einem Bereich von 1,0 bis 1,1 liegt.

2. Trageartikel nach Anspruch 1, wobei ein Dehnungsverhältnis der elastischen Hilfselemente (19) in einem Bereich von 1,5 bis 4,0 liegt.

3. Trageartikel nach Anspruch 1, wobei die Vielzahl der klebenden Verbindungspunkte (20) in einer Längsrichtung nahe der sich gegenüber liegenden Seitenkanten des Panels (25) angeordnet sind.

4. Trageartikel nach einem beliebigen der Ansprüche 1 bis 3, wobei jedes der elastischen Hilfselemente (19) in einem ungedehnten Zustand eine in der taillenumgebenden Richtung gemessene Größe aufweist, die im Wesentlichen einer in der taillenumgebenden Richtung gemessenen Größe des in den vorderen und hinteren Taillenbereichen liegenden Panels (25) entspricht.

## Revendications

1. Article vestimentaire jetable à porter (1) comprenant généralement une couche de base (2) définissant des régions de ceinture avant et arrière (7, 9) opposées l'une à l'autre et une région d'entrejambe (8) s'étendant entre lesdites régions de ceinture et un panneau (25) absorbant pour les liquides fixé à un côté intérieur de ladite couche de base (2) de manière à s'étendre au-dessus de ladite région d'entrejambe (8) plus loin dans lesdites régions de ceinture avant et arrière (7, 9), ladite couche de base (2) ayant une la partie d'extrémité supérieure (10) entourant la région de ceinture située à l'extérieur longitudinalement opposées (25a) dudit panneau et des zones latérales transversalement opposées (11) situées à l'extérieur transversalement par des bords latéraux opposés (25b) dedit panneau, dans lequel les régions de ceinture avant et arrière définies par ladite couche de base étant relié les uns aux autres au voisinage des bords latéraux (13) desdites zones latérales transversalement opposées (11) pour former un passage pour la taille (38) et une paire des ouvertures pour les jambes (39), et ladite partie d'extrémité supérieure entourant la région de ceinture étant pourvue d' éléments élastiques entourant la région de ceinture (15) de sorte que lesdites éléments élastiques entourant la région de ceinture sont contractile dans une direction circonférentielle de la taille, dans lequel :
ladite couche de base (2) comprend une première couche (6) faisant face audit panneau (25) et une deuxième couche (5) située à l'extérieur de ladite première couche, et une pluralité d' éléments élastiques auxiliaires (19) s'étendant dans la direction de la taille circonférentielle à travers dudit panneau (25) sont disposés entre lesdites éléments élastiques entourant la région de ceinture et lesdits ouvertures pour les jambes dans au moins une desdites régions de ceinture avant ou arrière (7, 9), et lesdits éléments élastiques auxiliaires (15) sont interposés entre ladite première couche (6) et ladite deuxième couche (5) et fixés solidaire aux couches (5, 6) de manière pour être contractible dans ladite direction circonférentielle de la taille,
et chacun desdits éléments élastiques auxiliaires (19) comporte des parties d'extrémité transversalement opposées (19a) se trouvant au voisinage desdites bords latéraux (13) desdites zones latérales opposées transversalement et fixée à au moins l'une desdites première ou deuxième couches (6, 5) et une partie intermédiaire (19b) s'étendant entre lesdites parties d'extrémité opposées et est séparée des première et deuxième couches,
une pluralité des points de liaison adhésive (20) servant à joindre lesdites première et deuxième couches (6, 5) sont distribués entre chaque paire desdits éléments élastiques auxiliaires (19) adjacents les uns aux autres à une distance déterminée dans la direction longitudinale aussi bien que dans la direction circonférentielle de la talle,
lesdites points de liaison adhésive (20) sont distribués sensiblement dans toute la surface de chaque région (17) des régions de ceinture avant et arrière (7, 9) qui sont occupées par le panneau (25);
un rapport d'une dimension d'une région de ladite couche de base (2), dans lequel lesdits éléments élastiques auxiliaires (19) sont définies en tant que lesdits éléments élastiques auxiliaires ont contracté à une dimension desdits éléments élastiques auxiliaires dans un état non contracté dans une direction circonférentielle de la taille est dans une gamme de 1,0 à 1,1.

2. Article vestimentaire selon la revendication 1, dans lequel un rapport d'étirage desdits organes élastiques auxiliaires (19) est dans une gamme de 1,5 à 4,0.

3. Article vestimentaire selon la revendication 1, dans lequel ladite pluralité de points de liaison adhésive (20) est disposée dans ladite direction longitudinale au voisinage desdits bords latéraux opposés dudit panneau (25).

4. Article vestimentaire selon l'une quelconque des revendications 1 à 3, dans lequel chacun desdits éléments élastiques auxiliaires (19) dans un état non étiré a une dimension mesurée dans ladite direction circonférentielle de la taille qui est sensiblement égale à une dimension dans ladite direction circonférentielle de la taille dudit panneau (25) mesurée dans lesdites régions de ceinture avant et arrière, tel que mesuré
